# EUROPEAN PATENT APPLICATION

(11) **EP 4 431 039 A1**
(43) Date of publication of application: **18.09.2024**
(21) Application number: 22891846.2
(22) Date of filing: 01.11.2022
(51) Int. Cl.: A61B 18/02

(54) **J-T SLOT POSITION-ADJUSTABLE CRYOABLATION NEEDLE**

(30) Priority: 11.11.2021 CN 202111329752
(71) Applicant: Accu Target Medipharma (Shanghai) Co., Ltd., Shanghai 201318 (CN)
(72) Inventor: YANG, Chi, Shanghai 201318 (CN); CHANG, Zhaohua, Shanghai 201318 (CN)
(74) Representative: EP&C
(86) International application number: PCT/CN2022/128872
(87) International publication number: WO 2023/083050

(57) **Abstract**

The present invention discloses a cryoablation needle with an adjustable J-T slot position, including: a vacuum wall, a J-T slot and a J-T slot adjusting apparatus, where the vacuum wall includes: a needle rod and an inner tube; the needle rod is provided with a needle tip at a distal end; the inner tube penetrates through the needle rod, and a cavity is formed between the inner tube and the needle rod; a first preset distance exists between a distal end of the inner tube and the distal end of the needle rod; the J-T slot penetrates through the inner tube; a distal end of the J-T slot is capable of being switched between at least two adjusting positions relative to the vacuum wall, the two adjusting positions including: a first adjusting position and a second adjusting position; in areas in which the vacuum wall is distributed in an axis direction of the vacuum wall, an area in which the cavity is located is a vacuum insulation area, and an area in which the first preset distance exists is a target area; the first adjusting position is located in the target area; and the second adjusting position is located in the vacuum insulation area. According to the present invention, the distal end of the J-T slot is switched between the at least two adjusting positions, such that a cooling rate is greatly increased.

## Description

### TECHNICAL FIELD

The present invention relates to the technical field of cryoablation, and in particular to a cryoablation needle with an adjustable J-T slot position.

### BACKGROUND

Cryoablation is a treatment approach that uses low temperature to destroy diseased tissues, which is considered to be an efficient and minimally invasive method to treat malignant tumors. The cryoablation is easy to operate, has few complications, and can effectively relieve pain. At the same time, ice balls formed by ablation have clear boundaries and are easy to observe, and lesions near large blood vessels or important organs can be safely ablated. The cryoablation may also adopt a multi-needle freezing manner, so that a wider ablation range is achieved, which is suitable for large lesions and irregular lesions.

In the process of cell freezing, ice crystals are firstly formed outside cells, which causes the concentration of extracellular solute to increase, resulting in a hypertonic environment, and water in the cells enters the outside of the cells, resulting in intracellular dehydration. The cells that lose water become shrunk and cell membranes are deformed, resulting in a "solution damage" in a high-concentration toxic environment. At the same time, ice crystals formed in the cells directly damage organelles and the cell membranes, causing further necrosis, commonly known as an "intracellular ice damage". The intracellular ice damage directly damages cell structures, and therefore is more destructive to the cells. Generally, the lower a cooling rate of the cells, the greater the probability of the "solution damage", and the higher the cooling rate, the easier it is to induce the "intracellular ice damage". Therefore, the higher cooling rate is generally pursued in the process of tumor cryoablation, which can kill tumors more thoroughly and greatly save surgery time.

The development of the cryoablation has undergone three stages. The first stage is a liquid nitrogen conveying and refrigeration technology, which conveys liquid nitrogen at -196°C to a needle tip of a cryoablation needle by a low driving pressure to achieve the purpose of cryoablation. Because a cold source of this technology completely relies on the liquid nitrogen, which is located in a main machine or liquid nitrogen barrel and has a long conveying distance from the needle tip, during the conveying process, only when a whole conveying pipeline reaches -196°C, the temperature of the needle tip can reach -196°C. Therefore, the cooling rate of liquid nitrogen refrigeration is the lowest in the prior art. The second stage is a direct throttling refrigeration technology, which uses the principle of "Joule Thompson Effect" (J-T for short), and conveys ultra-high pressure gas at room temperature to the J-T slot (a capillary tube that produces the J-T effect) inside the cryoablation needle to directly throttle to produce low temperature. The cooling rate of this technology is relatively the highest in the prior art. However, structures such as the J-T slot and a finned tube inside the needle tip may consume a part of cold, thus prolonging cooling time. In addition, the ultra-high pressure gas used is not highly popularized and is expensive, resulting in difficulty in the promotion of this technology. The third stage is a pre-cooled throttling refrigeration technology, the principle of which is to pre-cool normal industrial gas that is at room temperature by a supporting main machine, and then convey pre-cooled normal industrial gas to the J-T slot inside the cryoablation needle to produce ablation temperature that is lower than preset temperature by throttling. This technology solves the problem that gas sources are expensive and scarce. Furthermore, this technology combines a throttling refrigeration technology, and thus the cooling rate thereof is obviously higher than that of the liquid nitrogen refrigeration technology, but is still lower than that of the direct throttling refrigeration technology.

### SUMMARY

For problems existing in the prior art, the present invention provides a cryoablation needle with an adjustable J-T slot position, to solve the problem of low cooling rate in the prior art.

In order to resolve the above technical problem, the present invention is implemented through the following technical solutions:

The present invention provides a cryoablation needle with an adjustable J-T slot position, including a vacuum wall, a J-T slot and a J-T slot adjusting apparatus, where
the vacuum wall includes: a needle rod and an inner tube;
the needle rod is provided with a needle tip at a distal end;
the inner tube penetrates through the needle rod, and a cavity is formed between the inner tube and the needle rod, the cavity being capable of forming a vacuum ;
in an axis direction of the vacuum wall, a first preset distance exists between a distal end of the inner tube and the distal end of the needle rod; the distal end of the inner tube is an end of the inner tube close to the needle tip;
the J-T slot penetrates through the inner tube;
a distal end of the J-T slot is capable of being switched between at least two adjusting positions relative to the vacuum wall, the at least two adjusting positions including: a first adjusting position and a second adjusting position; the distal end of the J-T slot is an end of the J-T slot close to the needle tip;
in areas in which the vacuum wall is distributed in the axis direction of the vacuum wall, an area in which the cavity is located is a vacuum insulation area, and an area in which the first preset distance exists is a target area;
the first adjusting position is located in the target area;
the second adjusting position is located in the vacuum insulation area;
the J-T slot adjusting apparatus is configured to enable the distal end of the J-T slot to be switched between the at least two adjusting positions by adjusting a position of the distal end of the J-T slot;
when the distal end of the J-T slot is located at the first adjusting position, in the axis direction of the vacuum wall, a second preset distance exists between the distal end of the J-T slot and the needle tip, and the second preset distance at least ensures that an ice ball formed by freezing is wrapped around the needle tip, that is, a refrigerant fluid returns from the inside of the target area and the inside of the vacuum insulation area after being sprayed from the J-T slot, where the refrigerant fluid exchanges heat with substances outside the whole target area during the process of returning from the inside of the target area; and
when the distal end of the J-T slot is located at the second adjusting position, in the axis direction of the vacuum wall, a third preset distance exists between the distal end of the J-T slot and a distal end of the vacuum insulation area, and the third preset distance at least ensures that the refrigerant directly returns from the inside of the vacuum insulation area after being sprayed from the J-T slot. Only a relatively static refrigerant exists in the target area, which will not exchange heat with the substances outside the target area, that is, the refrigerant does not release any cold in the target area during a freezing process, the distal end of the vacuum insulation area being an end of the vacuum insulation area close to the needle tip.

Preferably, the J-T slot includes: a J-T slot straight section and a J-T slot spiral section; from the distal end to a proximal end of the J-T slot, the J-T slot straight section and the J-T slot spiral section are distributed in sequence;
the J-T slot adjusting apparatus includes: a first sliding block and a first sliding block guiding portion;
the first sliding block guiding portion is connected to the J-T slot straight section, and the first sliding block is fixedly connected to the first sliding block guiding portion;
the first sliding block, the first sliding block guiding portion and the J-T slot straight section are capable of being controlled to synchronously move in the axis direction, to switch the distal end of the J-T slot between the adjusting positions; and
a position of a proximal end of the J-T slot spiral section is fixed relative to the vacuum wall, and the J-T slot spiral section is capable of being tensioned or compressed with movement of the J-T slot straight section in the axis direction, the proximal end of the J-T slot spiral section being an end of the J-T slot serial section far away from the needle tip.

Preferably, the cryoablation needle with the adjustable J-T slot position further includes: a first sealing assembly, where the first sealing assembly is hermetically connected to a proximal end of the inner tube, the proximal end of the inner tube being an end of the inner tube far away from the needle tip; and
a dynamic seal is formed between the first sealing assembly and the first sliding block guiding portion.

Preferably, the first sealing assembly includes: a sealing apparatus and prolonged tube, where
a distal end of the prolonged tube is hermetically connected to the proximal end of the inner tube, the distal end of the prolonged tube being an end of the prolonged tube close to the needle tip; and
the sealing apparatus is hermetically connected to a proximal end of the prolonged tube, and a dynamic seal is formed between the sealing apparatus and a second sliding block guiding portion, the proximal end of the prolonged tube being an end of the prolonged tube far away from the needle tip.

Preferably, the J-T slot adjusting apparatus includes a push tube, a second sliding block and a second sliding block guiding portion; where
a distal end of the push tube is connected to a proximal end of the J-T slot, the distal end of the push tube being an end of the push tube close to the needle tip;
a proximal end of the push tube is of a closed structure;
the second sliding block guiding portion is arranged in the axis direction;
the second sliding block is connected to the proximal end of the push tube, and the second sliding block and the second sliding block guiding portion are capable of sliding relative to each other; and
the second sliding block, the push tube, and the J-T slot are capable of being controlled to synchronously move in the axis direction, to switch the distal end of the J-T slot between the adjusting positions.

Preferably, the cryoablation needle with the adjustable J-T slot position further includes: a second sealing assembly, where
a fixed seal is formed between the second sealing assembly and a proximal end of the second sliding block guiding portion; and
a dynamic seal is formed between the second sliding block guiding portion and the push tube through the second sealing assembly.

Preferably, the vacuum wall further includes: an outer tube and a gasket, where
the gasket is arranged between an outer wall of the distal end of the inner tube and an inner wall of the needle rod to form a sealed connection;
a distal end of the outer tube is hermetically connected to a proximal end of the needle rod, a proximal end of the outer tube is hermetically connected to a proximal end of the inner tube; the distal end of the outer tube is an end of the outer tube close to the needle tip, and the proximal end of the outer tube is an end of the outer tube far away from the needle tip;
an outer diameter of the outer tube is greater than an outer diameter of the needle rod, and an inner diameter of the outer tube is greater than an inner diameter of the needle rod;
from the distal end to the proximal end of the inner tube, the inner tube sequentially includes: an inner tube front section and an inner tube rear section; an outer diameter of the inner tube rear section is greater than an outer diameter of the inner tube front section; an inner diameter of the inner tube rear section is greater than an inner diameter of the inner tube front section; and
the inner tube front section penetrates through the needle rod; and the inner tube rear section penetrates through the outer tube.

Preferably, the cryoablation needle with the adjustable J-T slot position further includes: a temperature measuring wire, where
a distal end of the temperature measuring wire is a temperature measuring point; the distal end of the temperature measuring wire is an end of the temperature measuring wire close to the needle tip; and
the temperature measuring point is arranged at the distal end of the J-T slot, and used for measuring temperature at the distal end of the J-T slot.

Preferably, the cryoablation needle with the adjustable J-T slot position further includes: a spring and a clamping piece, where
one end of the spring is capable of moving synchronously with the distal end of the J-T slot, and is further connected to the clamping piece; the clamping piece is capable of entering and exiting from a clamped position;
the other end of the spring is fixed relative to the vacuum wall;
when the clamping piece is located at the clamped position, the spring is limited by the clamping piece to keep in a deformation state, and the distal end of the J-T slot is located at the second adjusting position;
the deformation state is a compression state or a tension state; and
when the clamping piece exits from the clamped position, the spring is capable of generating a restoring force for restoring from the deformation state to a natural state, and the restoring force is capable of driving the distal end of the J-T slot to enter the first adjusting position from the second adjusting position.

Preferably, the cryoablation needle with the adjustable J-T slot position further includes: a spring stop collar, where
when the distal end of the J-T slot is switched from the second adjusting position to the first adjusting position, the spring stop collar is configured to limit the furthest distance of the spring moving toward a distal end, and when the distal end of the spring is located at the spring stop collar, the distal end of the J-T slot is located at the first adjusting position.

Preferably, the clamping piece includes: a positioning pin and a C-shaped ring, where
the positioning pin is arranged on the C-shaped ring;
the C-shaped ring is wrapped around an outer wall with a fixed position relative to the vacuum wall, and capable of preventing the clamping piece from falling off radially; and
when the distal end of the J-T slot is located at the first adjusting position, the positioning pin is configured to keep the spring in the deformation state.

Compared with the prior art, the present invention has the following advantages:
(1) According to the cryoablation needle with the adjustable J-T slot position provided in the present invention, the distal end of the J-T slot is adjustable in position, and at least includes two adjusting positions, and freezing is started after the J-T slot is returned to the inside of the vacuum insulation area, so that all conveying pipelines at a main machine side and a cryoablation needle side can be pre-purged (cooled), and no cold is consumed at the target area during a pre-purging process. Therefore, all cold is used for cooling the conveying pipelines, and thus the rate of this conveying pipeline cooling process is the highest. Furthermore, no cold is released at the target area during the pre-purging process, so that there are no frosting and freezing phenomena in the target area, and then formal surgery can be performed directly.
(2) According to the cryoablation needle with the adjustable J-T slot position provided in the present invention, the distal end of the J-T slot is adjustable in position, and at least includes two adjusting positions, only the target area of the vacuum wall of the pre-purged cryoablation needle is not cooled, freezing is started after the J-T slot is returned to the inside of the target area, and all heat loads only come from the target area and tumor tissues outside the target area. Therefore, the cooling rate of this freezing process can be greatly increased.
(3) According to the cryoablation needle with the adjustable J-T slot position provided in the present invention, after a needling test procedure ends, a pre-purging mode can be kept enabled, the inside (the distal end of the J-T slot) of the vacuum insulation area is kept at the lowest temperature. Since the target area does not release any cold, operations such as puncturing, scanning and positioning can be performed, and then the cryoablation needle is adjusted to a freezing mode after the puncturing is in place. In this case, the inside of the target area is directly reduced from the normal temperature to the lowest temperature instantly. Therefore, ultimate rapid cooling of the formal surgery can be achieved.
(4) The cryoablation needle with the adjustable J-T slot position provided in the present invention is wide in application range, and can be applied to all existing cryoablation technologies: liquid nitrogen conveying and refrigeration technology, direct throttling refrigeration technology and pre-cooled throttling refrigeration technology. The cryoablation needle with the adjustable J-T slot position is not only applicable to percutaneous cryoablation instruments, but also applicable to cryoablation instruments of natural orifice transluminal surgery.

Certainly, implementing any product of the present invention does not necessarily need to simultaneously achieve all the advantages described above.

### BRIEF DESCRIPTION OF DRAWINGS

In order to explain the technical solutions in the embodiments of the present invention or in the prior art more clearly, the drawings used in the description of the embodiments or the prior art will be briefly introduced below. Obviously, the drawings in the following description are merely some embodiments of the present invention. For those of ordinary skill in the art, other drawings can be obtained based on these drawings without creative efforts.
FIG. 1 is a diagram of a J-T slot adjustment principle of a hard cryoablation needle with an adjustable J-T slot position according to an embodiment of the present invention;
FIG. 2 is a diagram of a J-T slot adjustment principle of a flexible cryoablation needle with an adjustable J-T slot position according to an embodiment of the present invention;
FIG. 3 is a diagram of a pre-purging mode of a mandrel-linkage J-T slot adjusting solution for a hard cryoablation needle according to a preferred embodiment of the present invention;
FIG. 4 is a diagram of a freezing mode of a mandrel-linkage J-T slot adjusting solution for a hard cryoablation needle according to a preferred embodiment of the present invention;
FIG. 5 is a schematic diagram of a vacuum wall of a flexible cryoablation tube according to a preferred embodiment of the present invention;
FIG. 6 is a diagram of a pre-purging mode of a mandrel-linkage J-T slot adjusting solution for a flexible cryoablation tube according to a preferred embodiment of the present invention;
FIG. 7 is a diagram of a freezing mode of a mandrel-linkage J-T slot adjusting solution for a flexible cryoablation tube according to a preferred embodiment of the present invention;
FIG. 8 is a diagram of a pre-purging mode of a push tube-linkage J-T slot adjusting solution for a hard cryoablation needle according to a preferred embodiment of the present invention;
FIG. 9 is a diagram of a core adjusting mechanism in a pre-purging mode of a push tube-linkage J-T slot adjusting solution for a hard cryoablation needle according to a preferred embodiment of the present invention;
FIG. 10 is a diagram of a freezing mode of a push tube-linkage J-T slot adjusting solution for a hard cryoablation needle according to a preferred embodiment of the present invention;
FIG. 11 is a diagram of a core adjusting mechanism in a freezing mode of a push tube-linkage J-T slot adjusting solution for a hard cryoablation needle according to a preferred embodiment of the present invention;
FIG. 12 is a diagram of a pre-purging mode of a push tube-linkage J-T slot adjusting solution for a flexible cryoablation needle according to a preferred embodiment of the present invention;
FIG. 13 is a diagram of a freezing mode of a push tube-linkage J-T slot adjusting solution for a flexible cryoablation needle according to a preferred embodiment of the present invention;
FIG. 14 is a schematic diagram of a sealing assembly according to a preferred embodiment of the present invention;
FIG. 15 is a schematic diagram of a sliding block according to a preferred embodiment of the present invention; and
FIG. 16 is a schematic diagram of a clamping piece according to a preferred embodiment of the present invention.

Description of reference signs: 1 - J-T slot,
11 - J-T slot straight section,
12 - J-T slot spiral section,
2 - vacuum wall,
21 - needle rod,
211 - needle tip,
22 - inner tube,
221 - inner tube front section,
222 - inner tube rear section,
23 - outer tube,
24 - gasket,
25 - target area,
26 - vacuum insulation area,
27 - spring stop collar,
28 - vacuum tee,
281 - tee connecting portion,
282 - tee bypass,
291 - vacuum connecting tube,
292 - vacuum hose,
293 - return gas connecting tube,
294 - shunt,
3 - mandrel,
4 - finned tube,
5 - sealing assembly,
51 - sealing ring,
52 - sealing slot,
53 - sealing press piece,
54 - prolonged tube,
6 - gas intake tube,
7 - gas return tube,
8 - sliding block,
81 - guiding tube,
82 - sliding block Positioning slot
83 - middle fixing hole,
84 - gas intake/return tube guiding hole,
9 - handle,
91 - handle positioning slot,
92 - bend preventing piece
10 - clamping piece,
101 - hand-held portion,
102 - positioning pin,
103 - C-shaped ring,
120 - spring,
13 - outer sleeve,
14 - temperature measuring wire,
141 - temperature measuring point,
15 - shunt tee,
151- central duct,
153 - gas intake channel,
17 - push tube,
171 - push tube thin section,
172 - push tube thick section,
174 - gas intake notch,
18 - sealing tube,
181 - sealing tube guiding section,
182 - sealing tube sealing section,
183 - sealing tube connecting section,
19 - sealing gasket.

### DESCRIPTION OF EMBODIMENTS

The technical solutions in embodiments of the present invention will be clearly and fully described in combination with the drawings of the embodiments of the present invention; it is obvious that the described embodiments are only a part of, and not all embodiments of, present invention. Based on the embodiments of the present invention, all other embodiments obtained by those of ordinary skill in the art without creative efforts should fall within the protection scope of the present invention.

In the description of the specification of the present invention, it should be understood that the term "upper portion", "lower portion", "upper end", "lower end", "upper surface", "lower surface" or the like indicates an orientation or positional relationship based on that shown in the drawings, which is merely for ease of description and simplicity of description, and is not intended to indicate or imply that the apparatus or element referred to must have a particular orientation, be constructed and operate in a particular orientation, and therefore cannot be construed as a limitation on the present invention.

In the description of the specification of the present invention, the terms "first" and "second" are used for descriptive purposes only, and cannot be construed as indicating or implying relative importance or implicitly indicating the number of technical features indicated. Therefore, a feature defined with "first" and "second" may explicitly or implicitly include one or more of the features.

In the description of the present invention, "a plurality of" means multiple, such as two, three, four or the like, unless specifically defined otherwise.

The technical solutions of the present invention will be described in detail below by specific embodiments. The following several specific embodiments may be mutually combined, and same or similar concepts or processes may not be repeatedly described in some embodiments.

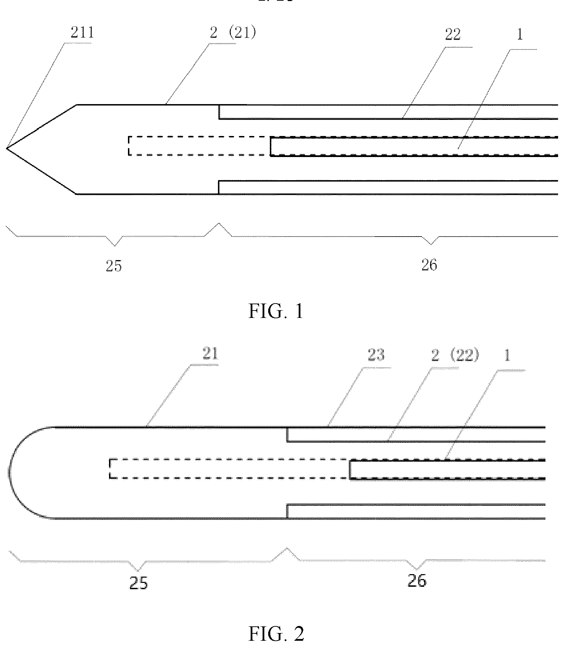

As shown in FIG. 1 and FIG. 2, which each is a diagram of a J-T slot adjustment principle of a cryoablation needle with an adjustable J-T slot position according to an embodiment of the present invention.

Referring to FIG. 1 and FIG. 2, the cryoablation needle with the adjustable J-T slot position according to this embodiment includes: a vacuum wall 2, a J-T slot 1 and a J-T slot adjusting apparatus.

The vacuum wall 2 includes: a needle rod 21 and an inner tube 22, and the needle rod 21 is provided with a needle tip 211 at a distal end. The inner tube 22 penetrates through the needle rod 21, and a cavity is formed between the inner tube 22 and the needle rod 21, the cavity being a cavity that can form a vacuum, which may be a permanent vacuum cavity or a real-time vacuum cavity. The vacuum cavity plays a role in heat insulation and preventing frostbite of normal tissues.

A first preset distance (the first preset distance can be understood as a spacing distance in an axis direction of the vacuum wall) exists between a distal end of the inner tube 22 and the distal end of the needle rod. The distal end of the inner tube 22 is an end of inner tube 22 close to the needle tip 211. The J-T slot 1 penetrates through the inner tube 22.

In areas in which the vacuum wall is distributed in the axis direction of the vacuum wall, since the vacuum cavity plays a role in heat insulation, an area in which the cavity is located is a vacuum insulation area 26, and an area in which the first preset distance exists is a target area 25.

The distal end of the J-T slot 1 has at least two adjusting positions, so that the distal end of the J-T slot can be switched between the at least two adjusting positions relative to the vacuum wall (for example, switching can be realized by moving in the axis direction of the vacuum wall), the at least two adjusting positions including: a first adjusting position and a second adjusting position. The distal end of the J-T slot 1 is an end of the J-T slot 1 close to the needle tip 211. When the first adjusting position is located in the target area, it can be understood as being in a freezing mode, as shown by dashed lines in FIG. 1. When the second adjusting position is located in the vacuum insulation area, it can be understood as being in a pre-purging mode, as shown by solid lines in FIG. 1.

The J-T slot adjusting apparatus is configured to enable the distal end of the J-T slot to be switched between the at least two adjusting positions, specifically, the distal end of the J-T slot can be adjusted in response to external manipulation, to be switched between the at least two adjusting positions. Furthermore, any apparatus that can realize this switching adjustment function does not depart from the description of this solution.

When the distal end of the J-T slot is located at the first adjusting position, a second preset distance exists between the distal end of the J-T slot and the needle tip, and the second preset distance at least ensures that an ice ball formed by freezing is wrapped around the needle tip. That is, a refrigerant fluid returns from the inside of the target area and the inside of the vacuum insulation area after being sprayed from the J-T slot, where the refrigerant fluid exchanges heat with substances outside the whole target area during the process of returning from the inside of the target area.

When the distal end of the J-T slot 1 is located at the second adjusting position, a third preset distance exists between the distal end of the J-T slot 1 and a distal end of the vacuum insulation area, and the third preset distance at least ensures that the refrigerant fluid directly returns from the inside of the vacuum insulation area after being sprayed from the J-T slot. Only a relatively static refrigerant exists in the target area, which will not exchange heat with the substances outside the target area. That is, the refrigerant does not release any cold in the target area during a freezing process. The distal end of the vacuum insulation area is an end of the vacuum insulation area close to the needle tip.

The first preset distance, the second preset distance and the third preset distance may be understood as spacing distances in the axis direction of the vacuum wall 2. In addition, an axial direction mentioned later can be understood as the axis direction of the vacuum wall 2.

In an embodiment, the vacuum wall is a hard-material vacuum wall, which can be applied to percutaneous cryoablation instruments. As shown in FIG. 1, the needle tip 211 is in the form of a pinpoint.

In an embodiment, the vacuum wall is a flexible-material vacuum wall, which can be applied to natural orifice transluminal ablation instruments. As shown in FIG. 2, the needle tip 211 is in the form of a circular arc. Preferably, the needle rod 21 and the inner tube 22 may be made of flexible nonmetallic materials or freely bendable metallic materials, such as PTFE or PTFE braided tubes or stainless-steel bellows.

In an embodiment, a use process of the cryoablation needle with the adjustable J-T slot position is as follows: Before surgery, the cryoablation needle in the pre-purging mode is taken out to be mutually connected to a main machine. The needle rod 21 (at least the target area 25) of the cryoablation needle is inserted into physiological saline. A needling test function is enabled. A rewarming operation is firstly performed during needling test. When the temperature of the needle tip rises to a certain temperature value within a certain time, it proves that a rewarming function is normal. Then, a program automatically performs a freezing operation. When the temperature of the needle tip is reduced to a certain temperature value within a certain time, it proves that a freezing function is normal. In this case, the time the needle tip is kept at the lowest temperature can be properly prolonged for sufficient pre-purging, and then the needling test is automatically stopped. During the freezing operation, whether there is a frosting phenomenon in the vacuum insulation area 26 is observed. If there is no frosting phenomenon, it proves that a heat insulation function is normal. Whether there is air leakage in the needle tip immersed in the physiological saline is observed during the whole process. If there is no air leakage, it proves that gas tightness is normal. After the needling test, conveying pipelines of both the main machine and the cryoablation needle have been pre-purged (cooled). Then, the freezing function can be enabled (or a separately configured pre-purging function can be enabled) at first, and freezing at this stage can be carried out at a lower working pressure, or gas can be intermittently introduced, so that the temperature at the distal end of the J-T slot can be kept at the lowest temperature while gas consumption can be reduced. Next, under the condition of keeping the freezing function enabled, percutaneous puncturing can be performed under the guidance of imaging, so that the needle tip can reach an expected tumor position. In this case, the J-T slot can be adjusted to move toward the distal end, and stop at the first adjusting position, to switch to the freezing mode. Since the whole conveying pipeline is already in a low temperature state, a cooling heat load of the cryoablation needle only exists in the target area 25 and tumor tissues outside the target area. Therefore, after switching to the freezing mode, the temperature of the distal end of the J-T slot can still be kept at the lowest temperature, and the outer wall of the target area 25 will be reduced from normal temperature to below -100°C instantly. In this way, surgical time for ablating a tumor with the same size is shortened, or a larger ablation range (ice ball) is produced within the same time. In addition, due to more rapid cooling of the tumor tissues, the probability of intracellular ice damage of tumor cells is greatly increased, and then freezing damage of the tumor cells is more thorough and the ablation effect is better.

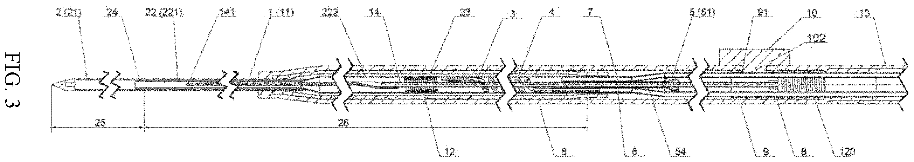

In a preferred embodiment, the position adjustment of the distal end of the J-T slot adopts an adjusting mode that a mandrel is in linkage with a J-T slot spring section. Refer to FIG. 3, FIG. 4, FIG. 6 and FIG. 7. Specifically, from the distal end to the proximal end of the J-T slot, the J-T slot sequentially includes: a J-T slot straight section 11 and a J-T slot spiral section 12, where the J-T slot straight section 11 and the J-T slot spiral section 12 may be of an integrated structure or a split connection structure, and may be made of different materials (for example, the J-T slot straight section 11 is made of stainless steel and the J-T slot spiral section 12 is made of nickel-titanium alloy) or the same material (such as stainless steel).

In an embodiment, the J-T slot adjusting apparatus may include: a mandrel 3 and a sliding block 8, where a distal end of the mandrel 3 is connected to a proximal end of the J-T slot straight section 11; the distal end of the mandrel 3 is an end of the mandrel 3 close to the needle tip 211, and the proximal end of the J-T slot straight section 11 is an end of the J-T slot straight section 11 far away from the needle tip 211; a proximal end of the mandrel 3 is connected to the sliding block 8; and the proximal end of the mandrel 3 is an end of the mandrel 3 far away from the needle tip 211. During the process of adjusting the position of the distal end of the J-T slot, the sliding block 8 is configured to slide in the axial direction, to drive the mandrel 3 to slide in the axial direction, and then drive the J-T slot straight section to slide. It can be seen that the sliding block 8, the mandrel 3 and the J-T slot straight section 11 can be controlled to synchronously move in the axis direction, and then, the distal end of the J-T slot straight section can be switched between the at least two adjusting positions. In this case, the position of a proximal end of the J-T slot spiral section 12 is unchanged (it can be understood as being fixed relative to the vacuum wall). The J-T slot spiral section 12 follows the sliding of the J-T slot straight section 11 by tensioning or compression. That is, the J-T slot spiral section can follow the movement of the J-T slot straight section 11 in the axis direction to be tensioned or compressed; and the proximal end of the J-T slot spiral section 12 is an end of the J-T slot spiral section 12 far away from the needle tip 211.

In the foregoing embodiment, the mandrel 3 is a first sliding block guiding portion. In this embodiment, the first sliding block guiding portion is connected to a component to be adjusted (J-T slot straight section), and the sliding block is connected to the first sliding block guiding portion. In a different embodiment, the sliding block 8 may be directly connected to the component to be adjusted (J-T slot straight section). The sliding block is arranged on an outer wall or an inner wall of the first sliding block guiding portion. The sliding block 8 is slidably connected to the first sliding block guiding portion. The first sliding block guiding portion is arranged in the axis direction of the vacuum wall, and used for guiding the sliding block 8 to move in the axis direction.

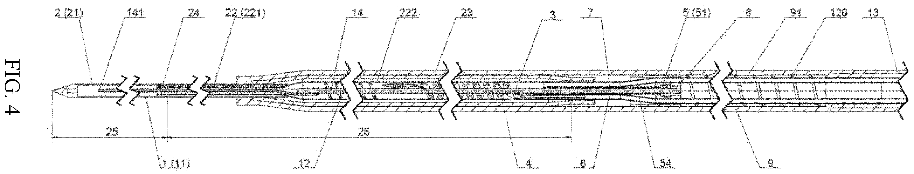

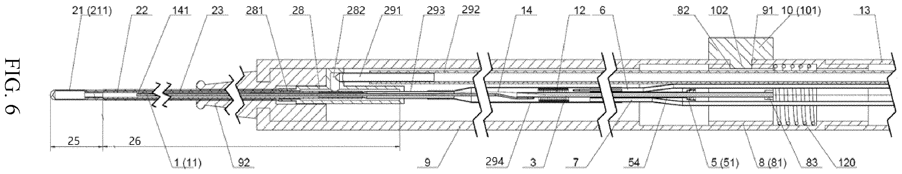

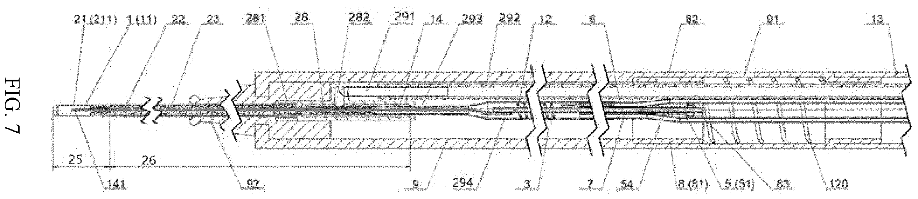

As shown in FIG. 3 and FIG. 6, the distal end of the J-T slot is located at the second adjusting position, that is, the diagram of the pre-purging mode. As shown in FIG. 4 and FIG. 7, the distal end of the J-T slot is located at the first adjusting position, that is, the diagram of the freezing mode.

In a different embodiment, the position of the distal end of the J-T slot can also be adjusted by the following structure: An elastic piece is separately arranged at the proximal end of the J-T slot, and in this case, a distal end of the mandrel is connected to the proximal end of the J-T slot. For the rest portion, refer to the foregoing embodiment, and details are not described here again.

In an embodiment, on the basis of the foregoing adjustment embodiment that the mandrel is in linkage with the J-T slot spring section, in order to prevent gas in the vacuum wall 2 from leaking during the process of adjusting the position of the distal end of the J-T slot, the cryoablation needle with the adjustable J-T slot position further includes a sealing assembly 5. Refer to FIG. 2 and FIG. 3. The sealing assembly 5 is arranged at a proximal end of the inner tube 22, and forms a dynamic seal with the J-T slot adjusting apparatus.

Furthermore, the sealing assembly 5 includes: a sealing ring 51, a sealing slot 52 and a sealing press piece 53. A distal end of the sealing slot 52 is hermetically connected to the proximal end of the inner tube 22. The sealing ring 51 is placed in the sealing slot 52. The sealing press piece 53 is screwed into the sealing slot 52 in the axial direction, to fix the sealing ring 51 between the sealing slot 52 and the sealing press piece 53. The mandrel 3 is inserted into the sealing ring 51 and the sealing press piece 53, so that the sealing ring 51 is radially extruded and deformed between the mandrel 3 and the sealing slot 52 to form a dynamic seal. Optionally, the sealing ring 51 may be a rubber sealing ring, such as a Buna-N rubber O-shaped ring, or may be a low-temperature-resistant Variseal sealing ring including fluoropolymer and a metal spring.

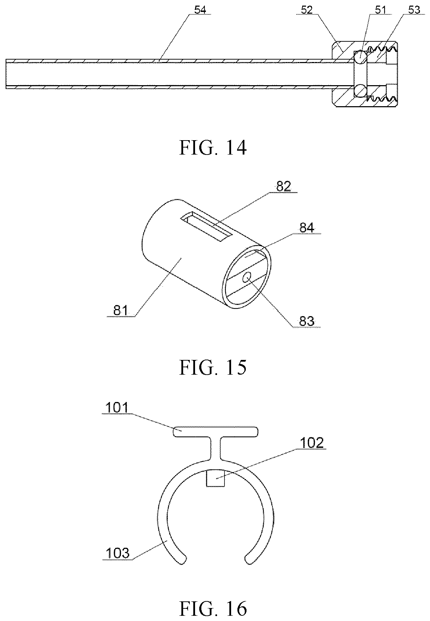

In a preferred embodiment, the sealing assembly 5 further includes: a prolonged tube 54. Refer to FIG. 14. The prolonged tube 54, a gas intake tube 6 and a gas return tube 7 are all inserted into the proximal end of the inner tube 2. The distal end of the sealing slot 52 is hermetically connected to a proximal end of the prolonged tube 54. For the connection mode of the sealing ring 51, the sealing slot 52 and the sealing assembly 53, refer to the description of the foregoing embodiment, and details are not described here again. The arrangement of the prolonged tube 54 helps to separate the sealing ring 51 from the proximal end of the cooler inner tube, the gas intake tube 6 and the gas return tube 7 at a certain distance to prevent sealing failure at low temperature.

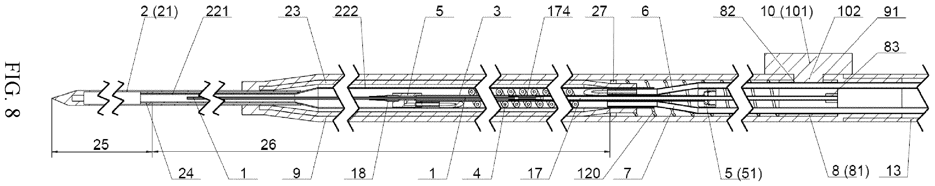

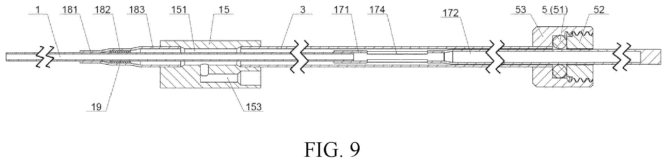

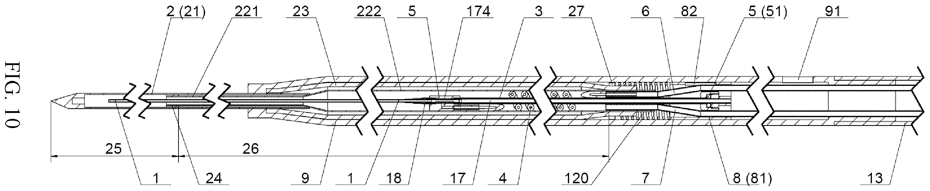

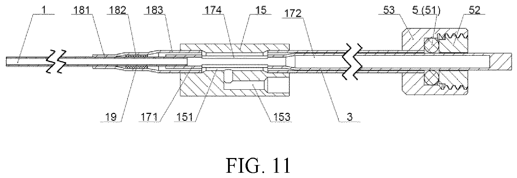

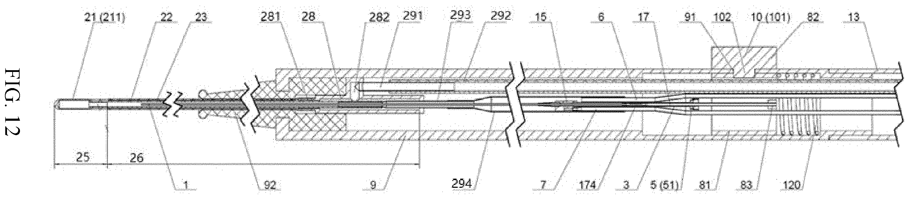

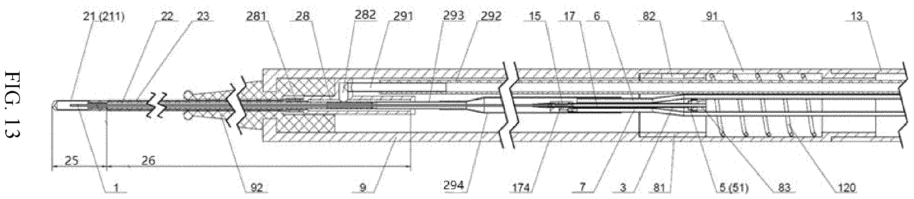

In a preferred embodiment, the position adjustment of the distal end of the J-T slot adopts an adjusting mode that a push tube is in linkage with the J-T slot. Refer to FIG. 8 to FIG. 13. Specifically, the J-T slot adjusting apparatus includes: a push tube 17 and a sliding block 8. A proximal end of the push tube 17 is of a closed structure (such as a blocked round tube), and the proximal end of the push tube 17 is connected to the sliding block 8. The proximal end of the push tube 17 is an end of the push tube 17 far away from the needle tip 211. A distal end of the push tube 17 is connected to a proximal end of the J-T slot 1. The distal end of the push tube 17 is an end of the push tube 17 close to the needle tip 211. During the process of adjusting the position of the distal end of the J-T slot, the sliding block 8 is configured to slide in the axial direction, to drive the push tube 17 to slide in the axial direction, and then drive the J-T slot 1 to slide, so as to realize the switching of the distal end of the J-T slot between the at least two adjusting positions. As shown in FIG. 8 and FIG. 12, the distal end of the J-T slot 1 is located at the second adjusting position, that is, the diagram of the pre-purging mode. As shown in FIG. 9, it is a diagram of a core adjusting mechanism in this mode. As shown in FIG. 10 and FIG. 13, the distal end of the J-T slot 1 is located at the first adjusting position, that is, the diagram of the freezing mode. As show in FIG. 11, it is a diagram of the core adjusting mechanism in this mode.

In a preferred embodiment, in order to improve a heat dissipation function, the cryoablation needle with the adjustable J-T slot position further includes: a finned tube 4. The finned tube 4 is arranged on an outer wall of the mandrel 3. Refer to FIG. 3, FIG. 4, FIG. 8 and FIG. 10. In addition, gas is introduced into the J-T slot 1 from the inside of the finned tube 4 or the inside of the gas intake tube 6 through a shunt tee 15. Refer to FIG. 9, FIG. 11, FIG. 12 and FIG. 13. The shunt tee 15 includes: a central duct 151 and a gas intake channel 153. The tee refers to two ends of the central duct 151 and the gas intake channel 153, respectively. A dynamic seal is formed between a distal end of the central duct 151 of the shunt tee 15 and the J-T slot 1, or a dynamic seal is formed between the distal end of the central duct 151 and the J-T slot 1 through a sealing tube 18 inserted into the distal end of the central duct 151.

In an embodiment, from a distal end to a proximal end of the sealing tube 18, the sealing tube 18 sequentially includes: a sealing tube guiding section 181, a sealing tube sealing section 182 and a sealing tube connecting section 183. Refer to FIG. 9 and FIG. 11. The sealing tube 18 is arranged between the J-T slot 1 and the vacuum wall 2. The sealing tube connecting section is inserted into the distal end of the central duct 151 and forms a fixed seal with the same. Furthermore, the cryoablation needle with the adjustable J-T slot position further includes: a sealing gasket 19. The sealing gasket 19 is located between the sealing tube sealing section 182 and the J-T slot 1. A low-temperature-resistant dynamic seal is realized by radially compressing the sealing tube sealing section 182. Sufficient gaps are provided between an outer wall of the J-T slot 1, an inner wall of the central duct 151 and an inner wall of the sealing tube connecting section 183 for ventilation. Preferably, the sealing gasket 19 is a PTFE tube.

In a preferred embodiment, the vacuum wall 2 further includes: an outer tube 23. Refer to FIG. 3, FIG. 4, FIG. 8 and FIG. 10. A distal end of the outer tube 23 is hermetically connected to a proximal end of the needle rod 21, and a proximal end of the outer tube 23 is hermetically connected to the proximal end of the inner tube 22.

In a preferred embodiment, in order to increase the internal volume of the proximal end of the inner tube, for example, the finned tube 4 may be inserted into the proximal end of the inner tube, or more other components can be accommodated. Since the internal volume of the proximal end of the inner tube needs to be increased, the internal volume of the proximal end of the vacuum wall also needs to be increased. An outer diameter of the outer tube 23 is greater than an outer diameter of the needle rod 21. An inner diameter of the outer tube 23 is greater than an inner diameter of the needle rod 21. The distal end of the outer tube 23 is an end of the outer tube 23 close to the needle tip 211. The proximal end of the outer tube 23 is an end of the outer tube 23 far away from the needle tip 211. Furthermore, from the distal end to the proximal end of the inner tube 22, the inner tube 22 sequentially includes: an inner tube front section 221 and an inner tube rear section 222. An outer diameter of the inner tube rear section 222 is greater than an outer diameter of the inner tube front section 221. An inner diameter of the inner tube rear section 222 is greater than an inner diameter of the inner tube front section 221. The inner tube front section 221 is located inside the needle rod 21. The inner tube rear section 222 is located inside the outer tube 23. Refer to FIG. 3, FIG. 4, FIG. 8 and FIG. 10.

In a preferred embodiment, the vacuum wall 2 further includes: a gasket 24. Refer to FIG. 3, FIG. 4, FIG. 8 and FIG. 10. The gasket 24 is arranged between the outer wall of the distal end of the inner tube 22 and the inner wall of the needle rod 21 to form a sealed connection.

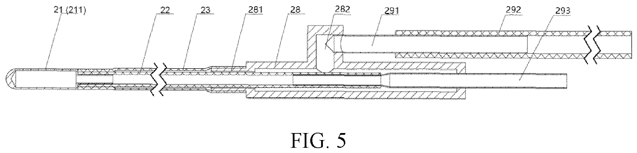

In a preferred embodiment, the vacuum wall of the flexible cryoablation needle may further include: a vacuum tee 28, a vacuum connecting tube 291, a vacuum hose 292 and a return gas connecting tube 293. Refer to FIG. 5. A proximal end of the inner tube 22 is connected to and sealed with a distal end of the return gas connecting tube 27. A proximal end of the outer tube 23 is connected to and sealed with a tee connecting portion 281. A proximal end of the vacuum tee 28 is connected to and sealed with the return gas connecting tube 27. A distal end of the vacuum connecting tube 291 is inserted into a tee bypass 282. The vacuum hose 292 is inserted into the vacuum connecting tube 291. By vacuum-pumping a proximal end of the vacuum hose 292, a gap between the inner tube 22 and the outer tube 23 can be kept in a vacuum state, to prevent frostbite of a normal natural cavity wall.

In an embodiment, the cryoablation needle with the adjustable J-T slot position further includes: a shunt 294, configured to seal gaps between the gas intake tube 6, the gas return tube 7 and the mandrel 3. The gas intake tube 6, the gas return tube 7 and the mandrel 3 are inserted into a proximal end of the shunt 294 for sealing. Refer to FIG. 6, FIG. 7, FIG. 12 and FIG. 13.

In a preferred embodiment, on the basis of the foregoing adjustment embodiment that the push tube is in linkage with the J-T slot, from the distal end to the proximal end of the push tube 17, the push tube 17 sequentially includes: a push tube thin section 171 and a push tube thick section 172. Refer to FIG. 4 to FIG. 7. A distal end of the push tube thin section 171 is fixed to the proximal end of the J-T slot 1. The push tube thin section 171 is provided with at least one gas intake notch 174, configured to insert the gas intake tube 6 of the J-T slot 1. A sufficient gap is provided between an outer wall of the push tube thin section 171 and an inner wall of the mandrel 3 for ventilation. A gap between an outer wall of the push tube thick section 172 and the inner wall of the mandrel 3 is small provided that the two only can move relative to each other. The push tube thick section 171 may be a solid round rod, or may be a round tube of which at least a proximal end is blocked.

In a preferred embodiment, on the basis of the foregoing adjustment embodiment that the push tube is in linkage with the J-T slot, in order to prevent gas in the vacuum wall 2 from leaking during the process of adjusting the position of the distal end of the J-T slot, the cryoablation needle with the adjustable J-T slot position also includes a sealing assembly 5. Refer to FIG. 4 to FIG. 7. The sealing assembly 5 forms a fixed seal with the proximal end of the mandrel 3, and forms a dynamic seal with the push tube 17.

Furthermore, the sealing assembly 5 includes: a sealing ring 51, a sealing slot 52 and a sealing press piece 53. A distal end of the sealing slot 52 is hermetically connected to the proximal end of the mandrel 3. The sealing ring 51 is placed in the sealing slot 52. The sealing press piece 53 is screwed into the sealing slot 52 in the axial direction, to fix the sealing ring 51 between the sealing slot 52 and the sealing press piece 53. The mandrel 3 is inserted into the sealing ring 51 and the sealing press piece 53, so that the sealing ring 51 is radially compressed and deformed between the mandrel 3 and the sealing slot 52 to form a dynamic seal. Optionally, the sealing ring 51 may be a rubber sealing ring, such as a Buna-N rubber O-shaped ring, or may be a low-temperature-resistant Variseal sealing ring including PTFE and a metal spring.

In a preferred embodiment, in order to better detect the freezing effect of the cryoablation needle, the cryoablation needle with the adjustable J-T slot position further includes: a temperature measuring wire 14. A distal end of the temperature measuring wire 14 is a temperature measuring point 141, and the distal end of the temperature measuring wire 141 is an end of the temperature measuring wire 141 close to the needle tip 211. Refer to FIG. 3, FIG. 4, FIG. 6 and FIG. 7. The temperature measuring point 141 is arranged at the distal end of the J-T slot 1, and used for measuring temperature at the distal end of the J-T slot 1. When being located in the target area, the distal end of the J-T slot 1 is configured to monitor the central temperature of a tumor during freezing and rewarming processes. When being located in the vacuum insulation area, the distal end of the J-T slot 1 is configured to indicate whether pre-purging is in place through the temperature during a pre-purging process. The temperature measuring wire 14 runs along the outer side of the J-T slot 1, and then leads out from the inside of the mandrel 3 or the push tube 17 to the outside, and glue is poured into the mandrel 3 or the push tube 17 for sealing. Preferably, the cryoablation needle with the adjustable J-T slot position further includes a rewarming wire. The position and arrangement of the rewarming wire are consistent with those of the temperature measuring wire, and the rewarming wire is used for achieving a rewarming function. Preferably, the temperature measuring wire and/or the rewarming wire is a T-type enameled thermocouple wire.

In an embodiment, position adjustment of the distal end of the J-T slot may be achieved by manual forward and backward adjustment, or may be achieved by a prefabricated spring 120, as shown in FIG. 3, FIG. 4, FIG. 6, FIG. 7, FIG. 8, FIG. 10, FIG. 12 and FIG. 13. The spring 120 is configured to keep the position of the distal end of the J-T slot. When the spring is in a natural state, the distal end of the J-T slot is located at the first adjusting position (freezing mode), as shown in FIG. 4, FIG. 7, FIG. 10 and FIG. 13. When the spring 120 is in a compression or tension state, the distal end of the J-T slot is located at the second adjusting position (pre-purging mode), as shown in FIG. 3, FIG. 6, FIG. 8 and FIG. 12. Furthermore, the cryoablation needle with the adjustable J-T slot position further includes a clamping piece 10, as shown in FIG. 3, FIG. 6, FIG. 8 and FIG. 12. When the distal end of the J-T slot is located at the first adjusting position, the clamping piece 10 is configured to keep the spring 120 in the compression or tension state. When the distal end of the J-T slot needs to be switched to the second adjusting position, only the clamping piece 10 needs to be pulled out.

As shown in FIG. 3, FIG. 6 and FIG. 12, the spring is in the compression state, one end (distal end) of the spring is connected to the sliding block 8 and the clamping piece 10, and the position of the other end (proximal end) of the spring is fixed relative to the vacuum wall. When the clamping piece 10 is clamped at a first position, the spring is kept in the compression state. In this case, the distal end of the J-T slot is located at the second adjusting position. When the distal end of the J-T slot needs to be adjusted to the first adjusting position, the clamping piece is pulled out of the first position. Under an elastic force of the spring, the sliding block 8 drives the J-T slot to slide towards the position of the distal end in the axis direction, and the J-T slot stops until sliding to the first adjusting position. In this case, the spring is in the natural state. Refer to FIG. 4, FIG. 7 and FIG. 13.

As shown in FIG. 8, the spring is in the tension state, one end (distal end) of the spring is connected to the sliding block 8 and the clamping piece 10, and the position of the other end (proximal end) of the spring is fixed relative to the vacuum wall. When the clamping piece 10 is clamped at the first position, the spring is kept in the tension state. In this case, the distal end of the J-T slot is located at the second adjusting position. When the distal end of the J-T slot needs to be adjusted to the first adjusting position, the clamping piece is pulled out of the first position. Under a resilience force of the spring, the sliding block 8 drives the J-T slot to slide towards the position of the distal end in the axis direction, and the J-T slot stops until sliding to the first adjusting position. In this case, the spring is in the natural state. Refer to FIG. 10.

In a preferred embodiment, in order to make the spring 120 in the natural state, that is, making the position of the distal end of the J-T slot at the first adjusting position more accurate, a spring stop collar 27 is further arranged. Refer to FIG. 8 and FIG. 10. The spring stop collar 27 is configured to prevent the spring 120 from further moving toward the distal end. When the distal end of the spring 120 is located at the spring stop collar 27, the distal end of the J-T slot 1 is located at the first adjusting position.

In an embodiment, the clamping piece 10 includes: a positioning pin 102, as shown in FIG 3. In this embodiment, the cryoablation needle with the adjustable J-T slot position further includes a handle 9. The handle 9 is arranged at a proximal end of the cryoablation needle, which is convenient for grasping. The handle 9 is provided with a handle positioning slot 91. The sliding block 8 is provided with a sliding block positioning slot 82. When the distal end of the J-T slot is located at the first adjusting position, the positioning pin 102 is synchronously inserted into the handle positioning slot 91 and the sliding block positioning slot 82, so that the position of the sliding block 8 is fixed relative to the handle 9, that is, the current pre-purging mode is kept. In this embodiment, the spring 120 is in the compression state in this case. When it needs to be switched to the freezing mode, only the positioning pin 102 needs to be pulled out of the handle positioning slot 91 and the sliding block positioning slot 82.

In an embodiment, in order to wrap around components such as the gas intake tube and the gas return tube, and make the cryoablation needle cleaner in appearance and more convenient in operation, an outer sleeve 13 is further arranged on an outer wall of the handle 9. Refer to FIG. 3, FIG. 4, FIG. 6, FIG. 7, FIG. 8. FIG. 10, FIG. 12 and FIG. 13.

In an embodiment, a bend preventing piece 92 is further arranged at a distal end of the handle of the flexible cryoablation needle, as shown in FIG. 6, FIG. 7, FIG. 12 and FIG. 13. The bend preventing piece 92 is arranged on an outer wall of the outer tube 23, which can protect a portion, which is in contact with the distal end of the handle, of the vacuum wall from bending.

In a preferred embodiment, in order to facilitate the fixing of the clamping piece and the insertion and removal adjustment of the clamping piece, the clamping piece 10 further includes: a hand-held portion 101 and a C-shaped ring 103. Refer to FIG. 16. The hand-held portion 101 and the positioning pin 102 are arranged on the C-shaped ring 103. The C-shaped ring 103 is wrapped around the outer wall of the handle 9, which can prevent the clamping piece from falling off radially.

In a different embodiment, when the handle 9 is not included, the C-shaped ring 103 only needs to be wrapped around an outer wall with a fixed position relative to the vacuum wall, which can also achieve the purpose of preventing the clamping piece from falling off radially.

In a different embodiment, when the distal end of the J-T slot is located at the first adjusting position, the spring can also be in the tension state. When the distal end of the J-T slot is located at the second adjusting position, the spring is in the natural state.

In a preferred embodiment, the sliding block 8 further includes: a guiding tube 81, a sliding block positioning slot 82, a middle fixing hole 83 and a gas intake/return tube guiding hole 84. Refer to FIG. 15. The guiding tube 81 is arranged in the axis direction of the vacuum wall. The sliding block positioning slot 82 is provided in an outer wall of the guiding tube 81, and is configured to insert the positioning pin of the clamping piece 10 in the foregoing embodiment, which may be omitted in a different embodiment when adjustment is performed without the use of a spring. The middle fixing hole 83 and the gas intake/return tube guiding hole 84 are provided in the guiding tube 81. The proximal end of the mandrel 3 is inserted into the middle fixing hole 83. The gas intake/return tube guiding hole 84 is configured for the gas intake tube 6 and the gas return tube 7 to penetrate through.

In the foregoing embodiment, the guiding tube 81 is a second sliding block and a second sliding block guiding portion. The guiding tube 81 can move in the axis direction. In a different embodiment, the mandrel arranged in the axis direction of the vacuum wall can also be used for guiding. The sliding block 8 is arranged on the outer wall of the mandrel, and can be guided to slide in the axis direction by sliding along the mandrel. In a different embodiment, the second sliding block guiding portion may be connected to the component to be adjusted (push tube). The sliding block is connected to the second sliding block guiding portion. The second sliding block guiding portion is arranged in the axis direction, and can move in the axis direction. The sliding block 8 drives the second sliding block guiding portion to move in the axis direction, and then drives the push tube to move in the axis direction.

In a preferred embodiment, the pre-purging mode, that is, a state that the distal end of the J-T slot 1 is located in the vacuum insulation area 26, can be set to a factory delivery state of the product, and an operator can directly complete the pre-purging of the product through a needling test procedure. After needling test/pre-purging is completed, the product is further adjusted to the freezing mode, that is, the distal end of the J-T slot 1 being located inside the target area 25. After the freezing mode is enabled, since the cryoablation needle is pre-purged, the target area 25 will rapidly cool down to the lowest temperature.

In the description of this specification, description of reference terms such as "an implementation", "an embodiment", "a specific implementation process" or "an example" means including specific features, structures, materials, or characteristics described in the embodiment or example in at least one embodiment or example of the present invention. In this specification, exemplary descriptions of the foregoing terms do not necessarily refer to the same embodiment or example. Furthermore, specific features, structures, materials or characteristics described may be combined in any suitable manner in one or more embodiments or examples.

Finally, it should be noted that the foregoing embodiments are merely intended for describing the technical solutions of the present invention, but not for limiting the present invention. Although the present invention is described in detail with reference to the foregoing embodiments, persons of ordinary skill in the art should understand that they may still make modifications to the technical solutions described in the foregoing embodiments or make equivalent replacements to some or all technical features thereof, without making the essence of the corresponding technical solutions departing from the scope of the technical solutions of the embodiments of the present invention.

## Claims

1. A cryoablation needle with an adjustable J-T slot position, comprising a vacuum wall, a J-T slot and a J-T slot adjusting apparatus, wherein
the vacuum wall comprises: a needle rod and an inner tube;
the needle rod is provided with a needle tip at a distal end;
the inner tube penetrates through the needle rod, and a cavity is formed between the inner tube and the needle rod, the cavity being capable of forming a vacuum ;
in an axis direction of the vacuum wall, a first preset distance exists between a distal end of the inner tube and the distal end of the needle rod; the distal end of the inner tube is an end of the inner tube close to the needle tip;
the J-T slot penetrates through the inner tube;
a distal end of the J-T slot is capable of being switched between at least two adjusting positions relative to the vacuum wall, the at least two adjusting positions comprising: a first adjusting position and a second adjusting position; the distal end of the J-T slot is an end of the J-T slot close to the needle tip;
in areas in which the vacuum wall is distributed in the axis direction of the vacuum wall, an area in which the cavity is located is a vacuum insulation area, and an area in which the first preset distance exists is a target area;
the first adjusting position is located in the target area;
the second adjusting position is located in the vacuum insulation area;
the J-T slot adjusting apparatus is configured to enable the distal end of the J-T slot to be switched between the at least two adjusting positions by adjusting a position of the distal end of the J-T slot;
when the distal end of the J-T slot is located at the first adjusting position, in the axis direction of the vacuum wall, a second preset distance exists between the distal end of the J-T slot and the needle tip, and the second preset distance at least ensures that an ice ball formed by freezing is wrapped around the needle tip; and
when the distal end of the J-T slot is located at the second adjusting position, in the axis direction of the vacuum wall, a third preset distance exists between the distal end of the J-T slot and a distal end of the vacuum insulation area, and the third preset distance at least ensures that a refrigerant directly returns from the inside of the vacuum insulation area after being sprayed from the J-T slot, the distal end of the vacuum insulation area being an end of the vacuum insulation area close to the needle tip.

2. The cryoablation needle with the adjustable J-T slot position according to claim 1, wherein the J-T slot comprises: a J-T slot straight section and a J-T slot spiral section; from the distal end to a proximal end of the J-T slot, the J-T slot straight section and the J-T slot spiral section are distributed in sequence;
the J-T slot adjusting apparatus comprises: a first sliding block and a first sliding block guiding portion;
the first sliding block guiding portion is connected to the J-T slot straight section, and the first sliding block is fixedly connected to the first sliding block guiding portion;
the first sliding block, the first sliding block guiding portion and the J-T slot straight section are capable of being controlled to synchronously move in the axis direction, to switch the distal end of the J-T slot between the adjusting positions; and
a position of a proximal end of the J-T slot spiral section is fixed relative to the vacuum wall, and the J-T slot spiral section is capable of being tensioned or compressed with movement of the J-T slot straight section in the axis direction, the proximal end of the J-T slot spiral section being an end of the J-T slot spiral section far away from the needle tip.

3. The cryoablation needle with the adjustable J-T slot position according to claim 2, further comprising: a first sealing assembly, wherein the first sealing assembly is hermetically connected to a proximal end of the inner tube; the proximal end of the inner tube is an end of the inner tube far away from the needle tip; and
a dynamic seal is formed between the first sealing assembly and the first sliding block guiding portion.

4. The cryoablation needle with the adjustable J-T slot position according to claim 3, wherein the first sealing assembly comprises: a sealing apparatus and a prolonged tube, wherein
a distal end of the prolonged tube is hermetically connected to the proximal end of the inner tube, the distal end of the prolonged tube being an end of the prolonged tube close to the needle tip; and
the sealing apparatus is hermetically connected to a proximal end of the prolonged tube, and a dynamic seal is formed between the sealing apparatus and the first sliding block guiding portion, the proximal end of the prolonged tube being an end of the prolonged tube far away from the needle tip.

5. The cryoablation needle with the adjustable J-T slot position according to claim 1, wherein the J-T slot adjusting apparatus comprises: a push tube, a second sliding block and a second sliding block guiding portion, wherein
a distal end of the push tube is connected to a proximal end of the J-T slot, the distal end of the push tube being an end of the push tube close to the needle tip;
a proximal end of the push tube is of a closed structure;
the second sliding block guiding portion is arranged in the axis direction;
the second sliding block is connected to the proximal end of the push tube, and the second sliding block and the second sliding block guiding portion are capable of sliding relative to each other; and
the second sliding block, the push tube, and the J-T slot are capable of being controlled to synchronously move in the axis direction, to switch the distal end of the J-T slot between the adjusting positions.

6. The cryoablation needle with the adjustable J-T slot position according to claim 5, further comprising: a second sealing assembly, wherein
a fixed seal is formed between the second sealing assembly and a proximal end of the second sliding block guiding portion; and
a dynamic seal is formed between the second sliding block guiding portion and the push tube through the second sealing assembly.

7. The cryoablation needle with the adjustable J-T slot position according to claim 1, wherein the vacuum wall further comprises: an outer tube and a gasket, wherein
the gasket is arranged between an outer wall of the distal end of the inner tube and an inner wall of the needle rod to form a sealed connection;
a distal end of the outer tube is hermetically connected to a proximal end of the needle rod, a proximal end of the outer tube is hermetically connected to a proximal end of the inner tube; the distal end of the outer tube is an end of the outer tube close to the needle tip, and the proximal end of the outer tube is an end of the outer tube far away from the needle tip;
an outer diameter of the outer tube is greater than an outer diameter of the needle rod, and an inner diameter of the outer tube is greater than an inner diameter of the needle rod;
from the distal end to the proximal end of the inner tube, the inner tube sequentially comprises: an inner tube front section and an inner tube rear section; an outer diameter of the inner tube rear section is greater than an outer diameter of the inner tube front section; an inner diameter of the inner tube rear section is greater than an inner diameter of the inner tube front section; and
the inner tube front section penetrates through the needle rod; and the inner tube rear section penetrates through the outer tube.

8. The cryoablation needle with the adjustable J-T slot position according to claim 1, further comprising: a temperature measuring wire, wherein
a distal end of the temperature measuring wire is a temperature measuring point; the distal end of the temperature measuring wire is an end of the temperature measuring wire close to the needle tip; and
the temperature measuring point is arranged at the distal end of the J-T slot, and used for measuring temperature at the distal end of the J-T slot.

9. The cryoablation needle with the adjustable J-T slot position according to any one of claims 1-8, further comprising: a spring and a clamping piece, wherein
one end of the spring is capable of moving synchronously with the distal end of the J-T slot, and is further connected to the clamping piece; the clamping piece is capable of entering and exiting from a clamped position;
the other end of the spring is fixed relative to the vacuum wall;
when the clamping piece is located at the clamped position, the spring is limited by the clamping piece to keep in a deformation state, and the distal end of the J-T slot is located at the second adjusting position;
the deformation state is a compression state or a tension state; and
when the clamping piece exits from the clamped position, the spring is capable of generating a restoring force for restoring from the deformation state to a natural state, and the restoring force is capable of driving the distal end of the J-T slot to enter the first adjusting position from the second adjusting position.

10. The cryoablation needle with the adjustable J-T slot position according to claim 9, further comprising: a spring stop collar, wherein
when the distal end of the J-T slot is switched from the second adjusting position to the first adjusting position, the spring stop collar is configured to limit the furthest distance of the spring moving toward a distal end, and when the distal end of the spring is located at the spring stop collar, the distal end of the J-T slot is located at the first adjusting position.

11. The cryoablation needle with the adjustable J-T slot position according to claim 9, wherein the clamping piece comprises: a positioning pin and a C-shaped ring, wherein
the positioning pin is arranged on the C-shaped ring;
the C-shaped ring is wrapped around an outer wall with a fixed position relative to the vacuum wall; and
when the distal end of the J-T slot is located at the first adjusting position, the positioning pin is configured to keep the spring in the deformation state.
